# EUROPEAN PATENT SPECIFICATION

(11) **EP 2 146 704 B1**
(45) Date of publication and mention of the grant of the patent: **23.03.2016**
(21) Application number: 08748930.8
(22) Date of filing: 16.04.2008
(51) Int. Cl.: A61K 31/167, A61K 31/57, A61P 11/06

(54) **USE OF A COMPOSITION COMPRISING FORMOTEROL AND BECLOMETASONE DIPROPIONATE FOR THE TREATMENT OF AN EXACERBATION OF ASTHMA**
VERWENDUNG EINER ZUSAMMENSETZUNG MIT FORMOTEROL UND BECLOMETHASON-DIPROPIONAT ZUR BEHANDLUNG EINES VERSCHLIMMERTEN ASTHMAZUSTANDES
UTILISATION D'UNE COMPOSITION COMPRENANT DU FORMOTÉROL ET DU DIPROPIONATE DE BÉCLOMÉTHASONE POUR LE TRAITEMENT DE L'ASTHME EXACERBE

(30) Priority: 19.04.2007 EP 07007930
(43) Date of publication of application: 27.01.2010
(60) Divisional application: 16152451.7
(73) Proprietor: CHIESI FARMACEUTICI S.p.A., 43100 Parma (IT)
(72) Inventor: CHIESI, Paolo, I-43100 Parma (IT); RONDELLI, Ivano, I-43100 Parma (IT); ACERBI, Daniela, I-43100 Parma (IT); POLI, Gianluigi, I-43100 Parma (IT)
(74) Representative: Minoja, Fabrizio
(86) International application number: PCT/EP2008/003012
(87) International publication number: WO 2008/128685

(56) References cited:
- WO-A-02/28368
- WO-A-02/28377
- WO-A-02/28378
- WO-A-99/64014
- RAZZETTI ET AL: "Formoterol and beclomethasone dipropionate interact positively in antagonising bronchoconstriction and inflammation in the lung" PHARMACOLOGICAL RESEARCH, ACADEMIC PRESS, LONDON, GB, vol. 55, no. 5, 13 April 2007 (2007-04-13), pages 426-432, XP022021221 ISSN: 1043-6618
- BOUROS D ET AL: "Formoterol and beclomethasone versus higher dose beclomethasone as maintenance therapy in adult asthma." THE EUROPEAN RESPIRATORY JOURNAL : OFFICIAL JOURNAL OF THE EUROPEAN SOCIETY FOR CLINICAL RESPIRATORY PHYSIOLOGY SEP 1999, vol. 14, no. 3, September 1999 (1999-09), pages 627-632, XP002449946 ISSN: 0903-1936
- Paul M. O'byrne ET AL: "Budesonide/Formoterol Combination Therapy as Both Maintenance and Reliever Medication in Asthma", American Journal of Respiratory and Critical Care Medicine, vol. 171, no. 2, 15 January 2005 (2005-01-15), pages 129-136, XP055122196, ISSN: 1073-449X, DOI: 10.1164/rccm.200407-884OC
- SINGH D ET AL: "Tolerability of high cumulative doses of the HFA modulite beclomethasone dipropionate/formoterol combination inhaler in asthmatic patients", PULMORNARY PHARMACOLOGY & THERAPEUTICS, ACADEMIC PRESS, GB, vol. 21, no. 3, 1 June 2008 (2008-06-01), pages 551-557, XP022649868, ISSN: 1094-5539, DOI: 10.1016/J.PUPT.2008.01.001 [retrieved on 2008-01-19]
- Jaro Ankerst ET AL: "Tolerability of a high dose of budesonide/formoterol in a single inhaler in patients with asthma", Pulmonary Pharmacology & Therapeutics, vol. 16, no. 3, 1 June 2003 (2003-06-01), pages 147-151, XP055156713, ISSN: 1094-5539, DOI: 10.1016/S1094-5539(03)00004-X
- PAPI ALBERTO ET AL: "Beclometasone-formoterol as maintenance and reliever treatment in patients with asthma: a double-blind, randomised controlled trial.", THE LANCET. RESPIRATORY MEDICINE MAR 2013, vol. 1, no. 1, March 2013 (2013-03), pages 23-31, ISSN: 2213-2619

## Description

### FIELD OF THE INVENTION

The present invention relates to the use of a composition according to claim 1 comprising a fixed combination of
a) formoterol, a pharmaceutically acceptable salt or solvate thereof or a solvate of such a salt; and
b) beclometasone dipropionate;
for the manufacture of a medicament for use in the treatment of an exacerbation of asthma, intermittent asthma and/or episodes in chronic asthma during the maintenance therapy of asthma with the same composition, for symptomatic relief, when needed.

### BACKGROUND OF THE INVENTION

Asthma is a disease which is becoming more prevalent and is the most common disease of childhood. It can be identified by recurrent wheeze and intermittent air flow limitation. Despite many advances in its understanding, asthma remains a poorly understood and often poorly treated disease. Previously, contraction of airway smooth muscles has been regarded as the most important feature of asthma. Recently there has been a marked change in the way asthma is managed, deriving from the fact that asthma is recognized as a chronic inflammatory disease. Uncontrolled airway inflammation may lead to mucosal damage and structural changes causing irreversible narrowing of the airways and fibrosis of the lung tissue. Therapy should therefore be aimed at controlling symptoms related to airway obstruction (e.g. wheezing, dyspnea) and at the same time provide basis for treating the underlying inflammation.

The acute asthma symptoms can be relieved by first generation beta-2 adrenoceptor agonists such as salbutamol, fenoterol and terbutalin (short-acting beta-2 agonists) or second generation ones such as formoterol and salmeterol (long-acting beta-2 agonists) which overcome the disadvantage of the short duration of action particularly for patients with nocturnal asthma.

Maintenance therapy is typically provided by anti-inflammatory glucocorticosteroids such as beclometasone dipropionate, budesonide, fluticasone propionate, mometasone furoate and ciclesonide. Recent therapeutic strategy is aimed at both controlling the symptoms and reducing the inflammation by combinations of a long-acting beta-2 agonist and a glucocorticosteroid.

Inhalation has become the primary route of administration in the treatment of asthma. Typical delivery systems for inhalable drugs are: pressurised metered dose inhalers, dry powder inhalers, or nebulizers (ultrasonic, jet, soft-mist etc.).

In any case, it is normal clinical practice to administer combination inhalers twice, sometimes once, daily at a dose related to the severity of asthma as a maintenance therapy and to use a short-acting beta-2 agonist, such as salbutamol or terbutaline, as required to relieve any breakthrough symptoms and acute situations, such as asthma exacerbations.

These acute situations may increase in frequency and severity with loss of disease control, requiring additional administration of short-acting beta-2 agonists.

Therapy with different medications and devices may lead to poor compliance to treatment from the patients, with consequent potential under-treatment and negative impact on their quality of life.

In particular the availability of a single inhaler for maintenance and rescue treatment may lead to better patient's compliance to treatment and asthma control.

In this direction WO 99/64014 proposes the use of a budesonide-formoterol combination for prevention or treatment of an acute condition of asthma. O' Byrne et al. (Am. J. Respir. Crit. Care Med., 171, 129-136, 2005, confirm the clinical efficacy of the combination budesonide/formoterol as both maintenance and reliever medication in asthma.

The present invention proposes an alternative combination featuring an enhanced efficacy and showing an excellent safety profile.

### SUMMARY OF THE INVENTION

The present invention provides the use of a suitable composition for the manufacture of a medicament for the rescue treatment of acute episodes as a complement to the maintenance treatment of asthma.

More specifically the present invention relates to the use of a composition comprising a fixed combination of
a) formoterol, a pharmaceutically acceptable salt or solvate thereof or a solvate of such a salt; and
b) beclometasone dipropionate; for the manufacture of a medicament for use in the treatment of an exacerbation of asthma, intermittent asthma and/or episodes in chronic asthma during the maintenance therapy of asthma with the same composition for symptomatic relief, when needed.

The use of the present composition, when needed, relates to the use during one or more of the following conditions:
i) an exacerbation of asthma or an acute condition of asthma is an acute asthma attack, when a patient on an irregular basis can be exposed to an agent i.e. pollen, a virus infection, cold air, exercise, perfumes or any other agent triggering an asthma attack;
ii) an intermittent asthma, i.e. in case of occasional (less than twice/week), brief exacerbations (hours to days), wherein nocturnal exacerbations are less than twice/month
iii) episodes, i.e. short periods of acute attacks of bronchospasms in chronic asthma.

### DETAILED DESCRIPTION OF THE INVENTION

According to the present invention a fixed combination of formoterol and beclometasone dipropionate may be administered in addition to the maintenance treatment of chronic asthma on a regular basis for the treatment of an exacerbation or acute condition of asthma, an intermittent asthma and/or episodes in chronic asthma.

Moreover a fixed combination of formoterol and beclometasone dipropionate may be administered in addition to the maintenance treatment of chronic asthma on a regular basis for the prevention of an exacerbation or acute condition of asthma, an intermittent asthma and/or episodes in chronic asthma.

In fact, in assessing the acute tolerability of high, cumulative doses of the combination formoterol and beclometasone dipropionate in comparison with formoterol alone or to placebo we have surprisingly found that formoterol alone caused significantly greater decrease in serum potassium level than the fixed combination or placebo.

Therefore the proposed combination, even when administered in doses largely in excess than the recommended clinical dose, as in case of a rescue treatment of asthma, and in particular in case of exacerbations during the maintenance therapy of asthma, is safer than formoterol alone as shown in Example 4.

During the maintenance therapy of asthma with a regular administration of the present composition, i.e. twice daily, in case of exacerbations the symptoms can be counteracted by using additional doses of the same composition, thus permitting to the additional glucocorticosteroid component (beclometasone dipropionate) to suppress as early as possible the enhanced airway inflammation and to the additional long-acting beta-2 agonist (formoterol) to immediately reduce the bronchial constriction, minimising the risk of too frequent dosing from different inhalers.

According to the present invention the administration of the combination formoterol/beclometasone dipropionate when needed reduces the severity of exacerbations and minimizes the difficulty of *a priori* predicting the best dosage regimen of glucocorticosteroid (low dose or high dose) and of the short-acting or long-acting beta-2 agonist to be used in separated inhalers.

The composition of the present invention comprises a fixed combination of formoterol and beclometasone dipropionate.

Formoterol, (+) N-[2-hydroxy-5-(1-hydroxy-2-((2-(4-methoxyphenyl)-1-methylethyl) amino) ethyl) phenyl] formamide, particularly in the form of its fumarate salt, is a bronchodilator used in the treatment of inflammatory or obstructive airways diseases. Formoterol can exist in four stereochemical forms. The present invention includes the individual stereoisomers, and in particular the (R, R)-enantiomer, as well as mixtures thereof and preferably its racemic mixture.

Formoterol may be present in the composition as free base or as a pharmaceutically acceptable salt. Pharmaceutically acceptable salt of formoterol include, for example, salts of inorganic acids such as hydrochloric, hydrobromic, sulfuric, phosphoric acids and of organic acids such as maleic, fumaric, tartaric, citric, benzoic, 4-methoxybenzoic, 2- or 4-hydroxybenzoic, 4-chlorobenzoic, p-toluenesulphonic, methanesulphonic, ascorbic, salicylic, acetic, succinic, lactic, tricarballylic, hydroxy-naphthalene-carboxylic, gluconic, oleic acids.

Formoterol or its salts may be present in the composition in a particular crystalline form. The preferred salt of formoterol is formoterol fumarate, particularly in the form of dihydrate.

Beclometasone dipropionate, (1-beta, 16-beta)-9-chloro-11, 17, 21-trihydroxy-16-methyl pregna-1, 4-diene-3, 20-dione 17, 21-dipropionate, is a well known anti-inflammatory glucocorticosteroid, used by inhalation as an antiasthmatic compound.

The molar ratio of formoterol or pharmaceutically acceptable salt thereof to beclometasone dipropionate in a fixed combination, calculated as formoterol to beclometasone dipropionate, is generally from 1:1 to 1:500, preferably from 1:1 to 1:100, more preferably from 1:1 to 1:60, even more preferably from 1:3 to 1:30, most preferably from 1:12 to 1:26 and the most preferred ratio is 1:12.8 or 1:25.6.

A recommended maximum daily dose of formoterol in combination with beclometasone dipropionate for adults is 24 microgram.

A recommended maximum daily dose of beclometasone dipropionate in combination with formoterol for adults is 400 microgram.

These doses are recommended to avoid the exposition of the patient to high levels of the active compounds. However, in the present invention it has been found that it is possible for the patient to administer this mixture as often as needed for a short period of time.

In particular, the daily dose of formoterol for adults, including maintenance therapy, may be as high as 168 microgram, preferably 100 microgram, and more preferably 84 microgram and even more preferably 72 microgram.

The daily dose of beclometasone dipropionate for adults, including maintenance therapy, may be as high as 5600, preferably 2800 microgram, more preferably 2400 microgram, even more preferably 1400 and most preferably 1200 microgram.

The dose regimen will depend on the severity of the disease if mild, moderate or severe asthma and on the patient's age, sex, weight etc.

The combination may be administered by inhalation orally or intranasally. The combination is preferably administered by a dry powder inhaler, a pressurized metered dose inhaler, or a nebuliser.

When the combination of the invention is formulated in the form of a dry powder composition to be administered by a dry powder inhaler, for example a single or a multi dose inhaler, both the active ingredients are in the form of micronized particles, preferably with a particle size of less than 10 micron.

The composition may comprise one or more suitable diluents or carriers such as lactose, dextran, mannitol or glucose. Preferably lactose is used, more preferably alpha-lactose monohydrate. Both the active ingredients of the combination of the invention and the diluent/carrier may be in a micronized form. Their mixture can optionally be subjected to agglomeration and/or spheronization.

Otherwise a coarse diluent/carrier may be added to the composition comprising the active ingredients of the combination and optionally a diluent/carrier in the form of micronized particles, to form an ordered mixture. Said ordered mixture may optionally contain an additive to promote the release of the active ingredients.

Suitable additives are substances with anti-adherent, glidant or lubricant properties. Magnesium stearate is a particularly preferred additive.

When the combination of the invention is formulated in the form of a pressurized metered dose inhaler, the active ingredients are preferably both completely dissolved in a liquid propellant mixture. The propellants which can be used include chlorofluorocarbons (CFCs), hydrocarbons or hydrofluorocarbons (HFAs). Especially preferred propellants are HFA 134a (1,1,1,2-tetrafluoroethane), HFA 227 (1,1,1,2,3,3,3-heptafluoropropane) or their mixtures. The active ingredients propellant mixtures are optionally used in combination with one or more other propellants and/or one or more additives such as cosolvents, for example ethanol, surfactants and/or one or more lubricant, antioxidant, stabilizing and or preserving agents such as an aqueous mineral acid and preferably 1M hydrochloric acid.

When the combination of the invention is formulated in the form of a pressurized metered dose inhaler, particularly preferred are solution formulations wherein the two active ingredients of the composition are dissolved in the propellant mixture. More preferably the propellant mixture comprises and/or consists of hydrofluorocarbons, such as HFA 134a, HFA 227 or their mixtures, a cosolvent, such as ethanol and an aqueous mineral acid, such as 1M hydrochloric acid, as a stabilizing agent.

Particularly preferred are HFA solution formulations (as the one of the following Examples 1, 2 and 4) which upon actuation of the pressurized metered dose inhaler, on evaporation of the propellant mixture, feature an average particle size of the two active ingredients equal or below 1.1 micrometer. These formulations are also referred herewith as extrafine formulations. Extrafine formulations assure a co-deposition in the lung areas of both the active ingredients particles. This co-deposition results in enhanced therapeutic bronchodilator effects and in a safer medicament.

In fact, due to its optimized particle distribution and increased deposition in the peripheral airways, the solution formulation combination of the present invention, allows a reduction in the glucocorticosteroid dose and a consequent reduction of its systemic exposure with respect to the marketed budesonide - formoterol dry powder inhaler formulations as described in WO 99/64014 (Examples 3-4). In fact according to GINA (Global Initiative for Asthma) international guidelines, Workshop Report Updated 2003, daily doses of 400 microgram of beclometasone dipropionate HFA (extrafine pressurized solution formulation) and 800 microgram of budesonide dry powder inhaler formulation are equivalent.

Therefore the availability of an extrafine HFA solution formulation of the combination of the present invention may further improve the risk/benefit ratio over the prior art formulation in case of multiple administrations of the combination such as in the rescue treatment of asthma, and in particular in case of exacerbations during the maintenance therapy of asthma.

Other features of the invention will become apparent in the course of the following descriptions of exemplary embodiments.

In the following examples micronization is carried out in a conventional manner such that the particle size range for each component is suitable for administration by inhalation.

### EXAMPLE 1

A pressurized metered dose inhaler solution formulation of the combination formoterol fumarate + beclometasone dipropionate contained formoterol fumarate dihydrate 6 microgram/dose (0.010% w/w based on the total weight of the formulation), beclometasone dipropionate 100 microgram/dose (0.172% w/w), ethanol 12% w/w as cosolvent and hydrochloric acid (1 M) 0.024% w/w as stabilizing agent and HFA 134a to 100% as the propellant. The formulation was packed in aluminium cans fitted with 50 microliters valves.

### EXAMPLE 2

A pressurized metered dose inhaler solution formulation of the combination formoterol fumarate + beclometasone dipropionate contained formoterol fumarate dihydrate 6 microgram/dose (0.008% w/w based on the total weight of the formulation), beclometasone dipropionate 200 microgram/dose (0.271% w/w), ethanol 12% w/w as cosolvent and hydrochloric acid (1M) 0.019% w/w as stabilizing agent and HFA 134a to 100% as the propellant. The formulation was packed in aluminium cans fitted with 63 microliters valves.

### EXAMPLE 3

A dry powder inhaler formulation of the combination formoterol fumarate + beclometasone dipropionate contained micronized formoterol fumarate dihydrate 6 microgram/dose (0,06% w/w based on the total weight of the formulation), micronized beclometasone dipropionate 100 microgram/dose (1% w/w), 989 microgram/dose of a preblend mixture (9,89% w/w) constituted of micronized lactose and magnesium stearate 98:2 w/w), 8904 microgram/dose of coarse alpha-lactose monohydrate having a particle size comprised between 212 and 355 micron (89,04% w/w).

### EXAMPLE 4

A study was performed to evaluate the tolerability of high, cumulative doses of the fixed combination formoterol/beclometasone dipropionate or formoterol compared to placebo when administered in asthmatic patients on regular treatment with the combination at the maximum recommended daily dose (6 microgram of formoterol/100 microgram beclometasone dipropionate, 2 puffs bid.: corresponding to a total daily dose of 24 microgram of formoterol and of 400 microgram of beclometasone dipropionate).

The study was a double blind clinical comparison study of ten puffs of the pressurized metered dose inhaler solution formulation of the combination of Example 1 (formoterol 6 microgram + beclometasone dipropionate 100 microgram) or formoterol (6 microgram) or placebo during the maintenance treatment of asthma with 2 puffs bid. of the combination formoterol/beclometasone dipropionate of Example 1.

Cumulative doses (10 puffs) were administered on 3 separated days in addition to the morning dose maintenance (2 puffs). Primary endpoint was serum potassium, assessed over a 12 hours period after the cumulative doses. In addition the effects of the treatment on the ECG (electrocardiogram), QTc (QT interval corrected of the heart's electrical cycle), blood pressure and heart rate were evaluated at regular intervals over a 12 hours period after dosing. Plasma lactate and glucose were determined over 3 hours after dosing.

Formoterol alone caused significantly greater decrease in serum potassium level than the fixed combination or placebo. While no significant differences in serum potassium parameters were observed between the fixed combination and placebo.

QTc, plasma lactate and the other vital signs values observed with the combination were not statistically different from those with formoterol alone.

In conclusion the administration of high cumulative doses of the combination formoterol/beclometasone dipropionate in asthmatic patients on maintenance treatment with the same combination do not significantly reduce their serum potassium levels differently from formoterol alone. Therefore the combination even when administered in doses largely in excess than the recommended clinical dose, such as in case of the rescue treatment of asthma, and in particular in case of exacerbations during the maintenance therapy of asthma, is safer than formoterol in asthmatic patients.

## Claims

1. Use of a composition comprising a fixed combination of
a) formoterol, a pharmaceutically acceptable salt or solvate thereof or a solvate of such a salt; and
b) beclometasone dipropionate;
for the manufacture of a medicament administered by inhalation by a dry powder inhaler, formulated in form of a dry powder composition comprising one or more suitable diluents or carriers, or by a pressurized metered dose inhaler wherein both the active ingredients (a) and (b) are completely dissolved in a liquid propellant mixture, for use for the rescue treatment of acute episodes of asthma attacks, when needed, as a complement to the maintenance treatment of asthma with the same medicament.

2. Use according to claim 1, wherein the molar ratio of (a) to (b), calculated as formoterol to beclometasone dipropionate, is from 1:1 to 1:500.

3. Use according to claim 2, wherein the molar ratio of (a) to (b), calculated as formoterol to beclometasone dipropionate, is from 1:1 to 1:100.

4. Use according to claim 3, wherein the molar ratio of (a) to (b), calculated as formoterol to beclometasone dipropionate, is from 1:3 to 1:30.

5. Use according to claim 4, wherein the molar ratio of (a) to (b), calculated as formoterol to beclometasone dipropionate, is 1:12.8.

6. Use according to claim 4, wherein the molar ratio of (a) to (b), calculated as formoterol to beclometasone dipropionate, is 1:25.6.

7. Use according to any one of claims 1 to 6 wherein the active ingredient (a) is formoterol fumarate dihydrate.

8. Use according to any one of claims 1 to 6 wherein the active ingredient (a) is the R,R-enantiomer of formoterol or pharmaceutically acceptable salt or solvate thereof or a solvate of such a salt.

9. Use according to any previous claim wherein the medicament comprises a daily dose of formoterol for adults, including maintenance therapy, as high as 72 microgram.

10. Use according to any previous claim wherein the medicament comprises a daily dose of beclometasone dipropionate for adults, including maintenance therapy, as high as 1200 microgram.

11. Use according to any one of claims 1 to 10 wherein when the medicament is formulated in form of a dry powder composition the one or more suitable diluents or carriers are selected from lactose, dextran, mannitol and glucose.

12. Use according to claim 11, wherein both the active ingredients (a) and (b) of the combination and the diluent/carrier are in a micronized form.

13. Use according to claim 12, wherein a coarse diluent/carrier may be added to the composition to form an ordered mixture.

14. Use according to claim 13, wherein said ordered mixture may contain an additive to promote the release of the active ingredients selected from substances with anti-adherent, glidant or lubricant properties.

15. Use according to any one of claims 1 to 10 wherein when the medicament is formulated in the form of a pressurized metered dose inhaler the liquid propellant mixture comprises HFA 134a (1,1,1,2-tetrafluoroethane), HFA 227 (1,1,1,2,3,3,3-heptafluoropropane) or their mixtures, in combination with one or more cosolvent, surfactant, lubricant, antioxidant, stabilizing and/or preserving agent.

16. Use according to claim 15, wherein the liquid propellant mixture comprises HFA 134a (1,1,1,2-tetrafluoroethane), ethanol as a cosolvent and an aqueous mineral acid as stabilising agent.

17. Use according to claim 16, wherein the aqueous mineral acid is 1M hydrochloric acid.

18. Use according to any one of claims 16 to 17 which upon actuation of the pressurized metered dose inhaler, on evaporation of the propellant mixture, features an average particle size of the two active ingredients equal or below 1.1 micrometer.

## Patentansprüche

1. Verwendung einer Zusammensetzung, umfassend eine Fixkombination von
a) Formoterol, einem pharmazeutisch annehmbaren Salz oder Solvat davon, oder einem Solvat von solch einem Salz, und
b) Beclomethasondipropionat,
für die Herstellung eines Medikaments, verabreicht durch Inhalation durch einen Trockenpulverinhalator, formuliert in Form einer Trockenpulverzusammensetzung, umfassend ein oder mehr geeignete Verdünnungsmittel oder Träger, oder durch einen druckbeaufschlagten Maßdoseninhalator, wobei die beiden aktiven Bestandteile (a) und (b) in einer flüssigen Treibmittelmischung vollständig aufgelöst sind, zur Verwendung für die Rettungsbehandlung akuter Episoden von Asthmaanfällen, wenn nötig, als Ergänzung für die Erhaltungsbehandlung von Asthma mit demselben Medikament.

2. Verwendung gemäß Anspruch 1, wobei das molare Verhältnis von (a) zu (b), berechnet als Formoterol zu Beclomethasondipropionat, von 1:1 bis 1:500 beträgt.

3. Verwendung gemäß Anspruch 2, wobei das molare Verhältnis von (a) zu (b), berechnet als Formoterol zu Beclomethasondipropionat, von 1:1 bis 1:100 beträgt.

4. Verwendung gemäß Anspruch 3, wobei das molare Verhältnis von (a) zu (b), berechnet als Formoterol zu Beclomethasondipropionat, von 1:3 bis 1:30 beträgt.

5. Verwendung gemäß Anspruch 4, wobei das molare Verhältnis von (a) zu (b), berechnet als Formoterol zu Beclomethasondipropionat, 1:12,8 beträgt.

6. Verwendung gemäß Anspruch 4, wobei das molare Verhältnis von (a) zu (b), berechnet als Formoterol zu Beclomethasondipropionat, 1:25,6 beträgt.

7. Verwendung gemäß einem der Ansprüche 1 bis 6, wobei der aktive Bestandteil (a) Formoterolfumaratdihydrat ist.

8. Verwendung gemäß einem der Ansprüche 1 bis 6, wobei der aktive Bestandteil (a) das R,R-Enantiomer von Formoterol oder ein pharmazeutisch annehmbares Salz oder Solvat davon oder ein Solvat eines solchen Salzes ist.

9. Verwendung gemäß einem der voranstehenden Ansprüche, wobei das Medikament eine tägliche Dosis von Formoterol für Erwachsene umfasst, einschließlich der Erhaltungstherapie, die 72 Mikrogramm beträgt.

10. Verwendung gemäß einem der voranstehenden Ansprüche, wobei das Medikament eine tägliche Dosis von Beclomethasondipropionat für Erwachsene umfasst, einschließlich Erhaltungstherapie, die 1200 Mikrogramm beträgt.

11. Verwendung gemäß einem der Ansprüche 1 bis 10, wobei, wenn das Medikament in Form einer Trockenpulverzusammensetzung formuliert ist, die ein oder mehr geeigneten Verdünnungsmittel oder Träger ausgewählt werden aus Laktose, Dextran, Mannit und Glukose.

12. Verwendung gemäß Anspruch 11, wobei sowohl die aktiven Bestandteile (a) und (b) der Kombination, als auch das Verdünnungsmittel/der Träger sich in mikronisierter Form befinden.

13. Verwendung gemäß Anspruch 12, wobei ein grobes Verdünnungsmittel/ein grober Träger der Zusammensetzung hinzugefügt werden kann, um eine geordnete Mischung zu bilden.

14. Verwendung gemäß Anspruch 13, wobei die geordnete Mischung ein Additiv enthalten kann, um die Freisetzung der aktiven Bestandteile zu fördern, ausgewählt aus Substanzen mit Antihaft-, Gleit- oder Schmiereigenschaften.

15. Verwendung gemäß einem der Ansprüche 1 bis 10, wobei, wenn das Medikament in der Form eines druckbeaufschlagten Maßdosierinhalators formuliert ist, die flüssige Treibmittelmischung HFA 134a (1,1,1,2-Tetrafluorethan), HFA 227 (1,1,1,2,3,3,3-Heptafluorpropan) oder ihre Mischungen, in Kombination mit ein oder mehr Cosolventien, Surfactants, Schmiermitteln, Antioxidationsmitteln, Stabilisatoren und/oder Konservierungsstoffen umfasst.

16. Verwendung gemäß Anspruch 15, wobei die flüssige Treibmittelmischung HFA 134a (1,1,1,2-Tetrafluorethan), Ethanol als ein Cosolvens und eine wässrige Mineralsäure als Stabilisator umfasst.

17. Verwendung gemäß Anspruch 16, wobei die wässrige Mineralsäure 1M Salzsäure ist.

18. Verwendung gemäß einem der Ansprüche 16 bis 17, welche nach Betätigung des druckbeaufschlagten Maßdoseninhalators, nach Verdampfung der Treibmittelmischung, eine durchschnittliche Partikelgröße der zwei aktiven Bestandteile gleich oder kleiner 1,1 Mikrometer aufweist.

## Revendications

1. Utilisation d'une composition comprenant une combinaison fixe :
a) de formotérol, d'un de ses sels ou d'un de ses solvates pharmaceutiquement acceptables ou bien d'un solvate d'un tel sel ; et
b) de dipropionate de béclométhasone ;
pour la préparation d'un médicament administré par inhalation via un inhalateur de poudre sèche, formulée sous la forme d'une composition de poudre sèche comprenant un ou plusieurs diluants ou supports acceptables, ou via un aérosol-doseur mis sous pression, les deux ingrédients actifs (a) et (b) étant complètement dissous dans un mélange de propulseurs liquides, pour le traitement de secours d'épisodes aigus de crises d'asthme, en fonction des nécessités, à titre de complément au traitement de fond de l'asthme avec le même médicament.

2. Utilisation selon la revendication 1, dans laquelle le rapport molaire de (a) à (b), calculé sous la forme du formotérol au dipropionate de béclométhasone s'élève de 1:1 à 1:500.

3. Utilisation selon la revendication 2, dans laquelle le rapport molaire de (a) à (b), calculé sous la forme du formotérol au dipropionate de béclométhasone s'élève de 1:1 à 1:100.

4. Utilisation selon la revendication 3, dans laquelle le rapport molaire de (a) à (b), calculé sous la forme du formotérol au dipropionate de béclométhasone s'élève de 1:3 à 1:30.

5. Utilisation selon la revendication 4, dans laquelle le rapport molaire de (a) à (b), calculé sous la forme du formotérol au dipropionate de béclométhasone s'élève à 1:12,8.

6. Utilisation selon la revendication 4, dans laquelle le rapport molaire de (a) à (b), calculé sous la forme du formotérol au dipropionate de béclométhasone s'élève à 1:25,6.

7. Utilisation selon l'une quelconque des revendications 1 à 6, dans laquelle l'ingrédient actif (a) est du fumarate de formotérol dihydraté.

8. Utilisation selon l'une quelconque des revendications 1 à 6, dans laquelle l'ingrédient actif (a) est l'énantiomère R,R de formotérol ou d'un de sels ou d'un de ses solvates pharmaceutiquement acceptables ou bien d'un solvate d'un tel sel.

9. Utilisation selon l'une quelconque des revendications précédentes, dans laquelle le médicament comprend une dose quotidienne de formotérol pour adultes, y compris dans le cas d'une thérapie de fond, aussi élevée que 72 µg.

10. Utilisation selon l'une quelconque des revendications précédentes, dans laquelle le médicament comprend une dose quotidienne de dipropionate de béclométhasone pour adultes, y compris dans le cas d'une thérapie de fond, aussi élevée que 1.200 µg.

11. Utilisation selon l'une quelconque des revendications 1 à 10, dans laquelle le médicament est formulé sous la forme d'une composition de poudre sèche dans laquelle lesdits un ou plusieurs diluants ou supports acceptables sont choisis parmi le lactose, le dextran, le mannitol et le glucose.

12. Utilisation selon la revendication 11, dans laquelle les deux ingrédients actifs (a) et (b) de la combinaison et les diluants/support sont présents sous une forme micronisée.

13. Utilisation selon la revendication 12, dans laquelle on peut ajouter un diluant/support à gros grain à la composition pour obtenir un mélange ordonné.

14. Utilisation selon la revendication 13, dans laquelle ledit mélange ordonné peut contenir un additif pour favoriser la libération des ingrédients actifs, choisi parmi des substances possédant des propriétés antiadhérentes, glissantes ou lubrifiantes.

15. Utilisation selon l'une quelconque des revendications 1 à 10, dans laquelle le médicament est formulé sous la forme d'un aérosol-doseur mis sous pression, le mélange de propulseurs liquides comprenant du HFA 134a (1,1,1,2-tétrafluoroéthane), du HFA 227 (1,1,1,2,3,3,3-heptafluoropropane) ou leurs mélanges, en combinaison avec un ou plusieurs cosolvants, un ou plusieurs agents tensioactifs, un ou plusieurs lubrifiants, un ou plusieurs antioxydants, un ou plusieurs stabilisateurs et/ou un ou plusieurs conservateurs.

16. Utilisation selon la revendication 15, dans laquelle le mélange de propulseurs liquides comprend du HFA 134a (1,1,1,2-tétrafluoroéthane), de l'éthanol à titre de cosolvant et un acide minéral aqueux à titre de stabilisateur.

17. Utilisation selon la revendication 16, dans laquelle l'acide minéral aqueux est de l'acide chlorhydrique 1 M.

18. Utilisation selon l'une quelconque des revendications 16 à 17, qui, dès l'activation de l'aérosol-doseur mis sous pression, lors de l'évaporation du mélange de propulseurs, présente une granulométrie moyenne des deux ingrédients actifs égale ou inférieure à 1,1 µm.
